# EUROPEAN PATENT SPECIFICATION

(11) **EP 3 406 229 B1**
(45) Date of publication and mention of the grant of the patent: **20.08.2025**
(21) Application number: 18182071.3
(22) Date of filing: 04.05.2015
(51) Int. Cl.: A61K 9/00, A61K 31/525, A61F 9/00, A61M 35/00

(54) **OPHTHALMIC TREATMENT SOLUTION DELIVERY DEVICES AND DELIVERY AUGMENTATION METHODS**
OPHTHALMISCHE BEHANDLUNGSLÖSUNGSABGABEVORRICHTUNGEN UND ABGABEVERSTÄRKUNGSVERFAHREN
DISPOSITIFS D'ADMINISTRATION DE SOLUTION DE TRAITEMENT OPHTALMIQUE ET PROCÉDÉS D'AUGMENTATION D'ADMINISTRATION

(30) Priority: 12.05.2014 US 201414275192
(43) Date of publication of application: 28.11.2018
(62) Divisional of application: 15793441.5
(73) Proprietor: Epion Therapeutics, Inc., Burlington, MA 01803 (US)
(72) Inventor: RUBINFELD, Roy S., Bethesda, MD Maryland 20817 (US); KORTEWEG, Wayne, N. Stonington, CT Connecticut 06359 (US)
(74) Representative: Dehns

(56) References cited:
- WO-A1-00/63079
- WO-A1-2013/148895
- WO-A1-2013/148896

## Description

### BACKGROUND

Collagen cross-linking is a treatment for multiple ophthalmic disorders. In some cases, collagen cross-linking may also be combined with other treatments to improve corneal strength or optical refraction, such as corneal ring segment inserts, topography-guided laser, and the like. Corrective lenses are normally required after these treatments for weakened corneas, but with smaller, more normalized prescriptions. Increased corneal symmetry allows for more comfortable contact lens wear, often of daily disposable lenses. Collagen cross-linking limits deterioration of vision, increases unaided and uncorrected vision, and may reduce the need for corneal transplantation. Collagen cross-linking may also have a role in stabilizing and "locking in" refractive effects of other procedures. WO 2013/148896 discloses a preparation device and method for buffing the corneal epithelium allows better penetration of the epithelium by riboflavin solution when subsequently applied to the eye; and a riboflavin solution for use in corneal strengthening treatment and the like has a higher concentration of riboflavin and may include other components for increasing corneal cross-linking.

### SUMMARY

The invention discloses a preparation sponge for use in manipulation of tissue on the surface of an eye comprising: a tissue preparation surface, shaped for manipulating and rubbing across the surface of an eye; wherein at least the tissue preparation surface of the preparation sponge is rounded; and has no sharp edges. The preparation sponge has little or no risk of disrupting or perforating the epithelium. In some embodiments, the preparation sponge further comprises a handle operatively connected to the preparation sponge. In some embodiments, the preparation sponge may be a spherical or part spherical sponge without a handle, and may be held by forceps or similar instruments during preparation of the eye surface. In some embodiments, the preparation sponge is of part circular shape. In some embodiments, the preparation sponge may have a straight edge or other shaped edge attached to the handle. In some embodiments, the preparation sponge is made of a cellulose sponge material. In some embodiments, the preparation sponge is made of polyvinyl acetate (PVA) sponge material. In some embodiments, the preparation sponge is wetted with an ophthalmic solution prior to use in preparation of the eye surface. In some embodiments, the ophthalmic solution is 0.2% to 10.0% by weight riboflavin in an aqueous carrier, and optionally, sodium iodide, catalase, artificial tears, or any combinations thereof.

Use of the preparation sponge followed by a second sponge device or loading sponge may augment or enhance delivery of any ophthalmic treatment solution to the eye for use in photochemical treatment of the cornea or for other types of treatment, such as application of eye medications such as glaucoma medications or anti-inflammatory medications such as steroids. In certain embodiments, the treatment solution is a riboflavin solution.

Disclosed herein, in certain embodiments, is a preparation sponge that improves the passage or penetration of riboflavin or other ophthalmic drugs through the epithelial barrier to avoid the surgical complications that arise from de-epithelialization, which can result in more disruption of the tissue than is necessary for the procedure to be effective. The reduction in patient discomfort combined with more rapid restoration of visual acuity make a trans-epithelial procedure better for the patient. In some embodiments, the preparation sponge is sterile. In some embodiments, the preparation sponge is part circular shape when dry and is configured to absorb liquid and expand into a spherical or part-spherical shape when wetted. In some embodiments, the preparation sponge is spherical or part-spherical in its dry condition. The preparation sponge may be of cellulose, PVA, or urethane sponge material. The preparation sponge has little or no risk of disrupting or perforating the epithelium, resulting in as little disruption of the epithelium as possible while still increasing penetration of the riboflavin or other solution into the deeper layers of the cornea without disrupting or causing any significant epithelial defects in the corneal surface.

In some embodiments, the preparation sponge is of sponge or other sponge-like materials designed to enhance epithelial permeability by gently removing lipids, mucus, and dead surface epithelial cells. In some embodiments, the preparation sponge is packaged along with a blunt plastic shaft or other tool to assist in "twirling" this sponge around on the cornea or sclera or other parts of the eye to manipulate the tissue to be treated in an effort to enhance penetration of the riboflavin or other solution into the eye.

In various embodiments, the preparation sponge is used in one or more microsurgical ophthalmic procedures for tissue manipulation and management of fluids. In other embodiments, the preparation sponge is used in one or more microsurgical ophthalmic procedures for the management of one or more fluids. In some embodiments, the preparation sponge is placed on the cornea to moisten the cornea during one or more microsurgical procedures.

The above preparation sponge may be used in various microsurgical ophthalmic procedures, including preparation of the epithelium for loading of one or more fluids through the epithelium into the eye for photochemical or other treatment.

Other features and advantages of the present disclosure will become more readily apparent to those of ordinary skill in the art after reviewing the following detailed description and accompanying drawings.

### BRIEF DESCRIPTION OF THE DRAWINGS

The details of the present invention may be gleaned in part by study of the accompanying drawings, in which like reference numerals refer to like parts, and in which:
FIG. 1 exemplifies an enlarged perspective view of one embodiment of a preparation or tissue manipulating sponge device for use in preparing the corneal epithelium for subsequent application of an ocular treatment solution to the eye;
FIG. 2 is a cross section on the lines 2-2 of FIG. 1;
FIG. 3A is a top plan view of a spear shaped sponge attached to the handle of FIG. 1 and 2 prior to cutting the sponge into the rounded shape of FIG. 1 and 2, according to one example of a method of making the preparation sponge device of FIG. 1 and 2, in which the method of making the preparation sponge device does not form part of the invention;
FIG. 3B is a top plan view of a sponge blank attached to the handle of FIG. 1 and 2 prior to cutting the sponge into the rounded shape of FIG. 1 and 2, according to another example of a method of making the preparation sponge device of FIG. 1 and 2, in which the method of making the preparation sponge device does not form part of the invention;
FIG. 4 is a cut away top perspective view of the tissue preparation sponge device of FIG. 1 to 3, showing the sponge head with part of the handle cut away, with the sponge head in a dry condition;
FIG. 5 is a side elevation view of the preparation sponge device of FIG. 1 to 4, with the handle partially cut away as in FIG. 2 and 4;
FIG. 6 is a top end perspective view of the preparation sponge device of FIGS. 1 to 5, with the handle partially cut away as in FIGS. 4 and 5;
FIGS. 7 to 9 are cut away views of the preparation sponge device corresponding to the views of FIGS. 4 to 6 but with the sponge head in a wet, expanded condition;
FIG. 10 illustrates an exemplary use of the sponge of FIG. 1 to 9 to manipulate the corneal surface in a swirl action;
FIGS. 11A to 11C are top plan views illustrating some other embodiments of the preparation sponge with a sponge head of circular shape;
FIG. 12 is a top plan view illustrating another embodiment of the preparation sponge device having a spherical sponge head;
FIG. 13 is a perspective view illustrating a second sponge device or loading sponge placed on an eye for holding and loading of an ophthalmic treatment solution, which does not form part of the invention;
FIG. 14 is a vertical cross-sectional view illustrating the second sponge device of FIG. 13 following the curvature of most of the eye surface, with details of the eye itself omitted, which does not form part of the invention;
FIG. 15 is a perspective view similar to FIG. 13 illustrating a round loading sponge, which does not form part of the invention; and
FIG. 16 illustrates tabulated examples of corneal saturation or loading times (in minutes) for a 0.1% riboflavin solution and a 0.5% riboflavin solution when the epithelium is prepared or manipulated with the preparation sponge device of FIGS. 1 to 9 and one of the solution holding or loading sponges of FIG. 13 to 15 is used to load the riboflavin treatment solution. The term "micrometer(s)" is the SI units corresponding to the non-SI term "micron(s)" mentioned herein, i.e. one micrometer corresponds to one micron.

### DETAILED DESCRIPTION

A popular treatment of corneal diseases, including keratoconus, post-LASIK ectasia, and pellucid marginal degeneration, involves the removal of the epithelium followed by administration of a riboflavin solution and irradiation by ultraviolet-A light. Riboflavin acts as a photosensitizer and facilitates the cross-linking of stromal collagen fiber which prevents further disease progression. However, the removal of the epithelium carries numerous risks for the patient, including postoperative pain, infection risk, delayed wound healing, corneal perforation, stromal haze, and herpetic keratitis. Therefore, it would be safer to treat the patient without having to surgically remove the epithelium. Unfortunately, there are many obstacles to this approach because the intact epithelium prevents the cornea from rapidly and conveniently absorbing the riboflavin solution. This invention discloses novel devices and methods for allowing convenient treatment of corneal diseases with medications or with ophthalmic solutions such as riboflavin solutions without removing the epithelium, and may increase the effectiveness of such treatments.

After reading this description it will become apparent to one skilled in the art how to implement the disclosure in various alternative embodiments and alternative applications within the scope of the amended claims.

The time period for sufficient riboflavin to penetrate into the cornea when the solution is applied in drops to the eye without removal or treatment of the epithelium layer can be one to three hours. This is a problem for both patients undergoing treatment and for surgeons, in view of the extended time period needed. In some embodiments described below, eye surface preparation or manipulation reduces the initial time needed for an ophthalmic solution (e.g., a riboflavin solution or other ophthalmic solution) to penetrate sufficiently into the cornea in as little as seven to eleven minutes without requiring removal of the epithelium, significantly reducing patient discomfort and the time needed to complete the treatment.

Manipulation of the epithelium prepares it for improved penetration of the epithelium by the ophthalmic solution (e.g., a riboflavin solution), without having to remove the epithelium altogether. The cornea absorbs the riboflavin well until the corneal stroma is sufficiently loaded.

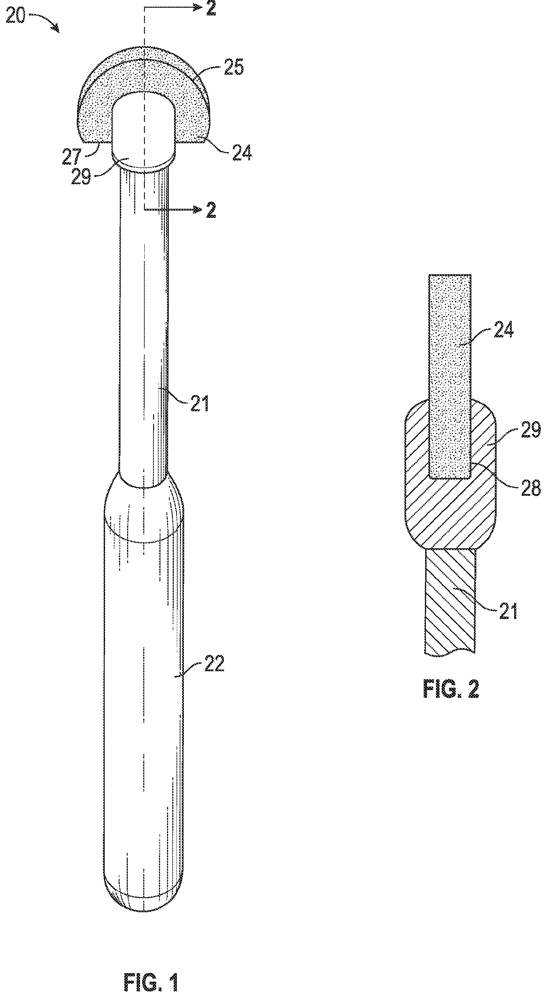

FIGS. 1 to 10 illustrate a preparation sponge for improving the penetration of riboflavin or other ophthalmic solutions or fluids through the epithelial barrier, while avoiding the surgical complications that arise from deepithelialization. The reduction in patient discomfort, the more rapid restoration of visual acuity, and the reduced risk of infection or stromal haze make a trans epithelial procedure better for the patient.

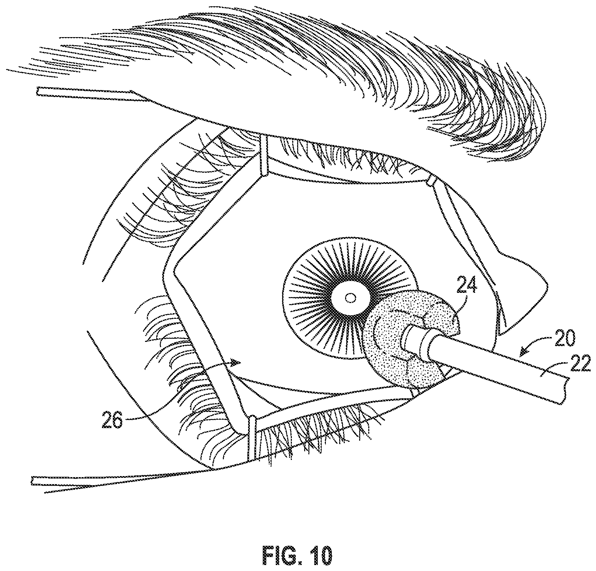

In some embodiments, a preparation sponge device 20 as illustrated in FIG. 1-9 is rubbed gently over the surface of the eye 26 after application of a topical anesthetic, as illustrated in FIG. 10. The preparation sponge 20 may be rubbed gently in a circular or swirling manner, in order to manipulate the eye surface, in other words to "buff" or "polish" the eye to a dull sheen. In one embodiment, the preparation sponge device 20 comprises an elongate handle or shaft 21 with a gripping portion 22 at one end and a sponge head 24 of relatively rigid sponge material attached to the other end of shaft 21, as illustrated in FIG. 1. When dry, the head 24 of this embodiment is a generally flat, part-circular or part disk shaped piece of surgical or medical grade sponge material such as relatively rigid, highly absorbent natural cellulose or highly absorbent, fast wicking polyvinyl acetate (PVA) material or the like, for example Ultracell^{®} PVA or other sponge materials used in Weck-Cel^{®} fluid control medical sponges as manufactured by Beaver-Visitec of Waltham, MA. The handle shaft 21 may be of any suitable material, such as injection molded plastic material, and may be transparent or opaque.

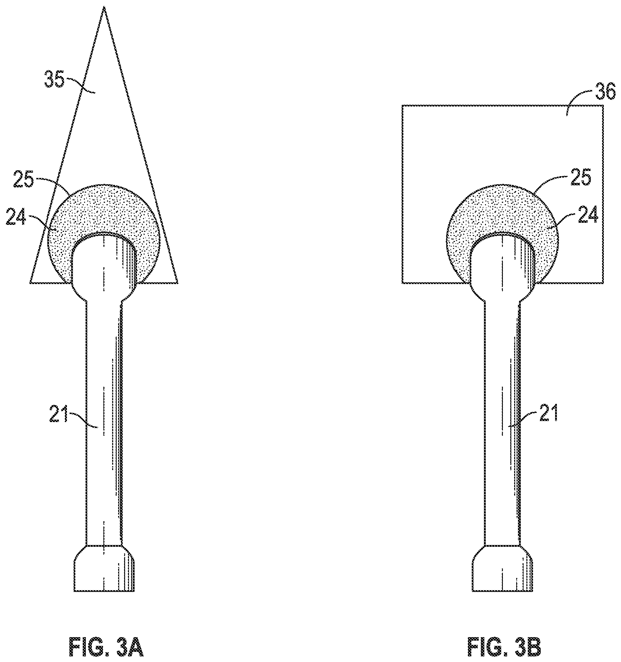

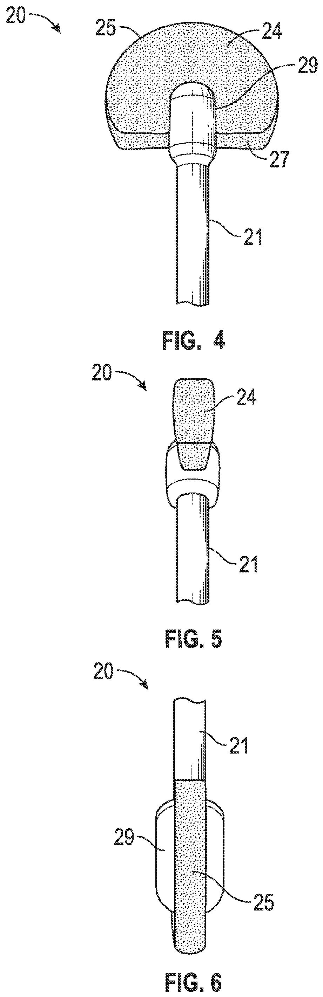

Sponge 24 has a rounded edge or rim 25 and a straight edge or rim 27, and is held at the center of the straight edge in recess 28 between spaced claws or end portions 29 of the shaft 21, as illustrated in FIG. 2. Sponge 24 may be secured in recess 28 via adhesive or the like. FIGS. 3A and 3B illustrate some alternative methods for manufacture of the preparation sponge device 20, which does not form part of the invention. The manufacturing method may be similar to that for conventional spear shaped medical sponges. In FIG. 3A, a spear shaped sponge 35 is secured to handle 21, and sponge 35 is then cut along line 25 to form part circular sponge 24. The thusformed rounded edge or rim 25 forms more than half of the periphery of a circle, as can be seen in FIG. 3A. In FIG. 3B, a sponge blank 36 is attached to handle 21, and is cut along line 25 to form the part-circular sponge 24. In each case, the rounded edge 25 passes the half way or semi-circle point and starts to curve back in towards the handle, as illustrated in FIG. 1 and 4 . The thickness of the sponge may be around 1 mm while the diameter of the part circular sponge may be in the range from 4.5 mm to around 8 mm. In one example, the diameter of the part-circular edge was about 6. 5 mm.

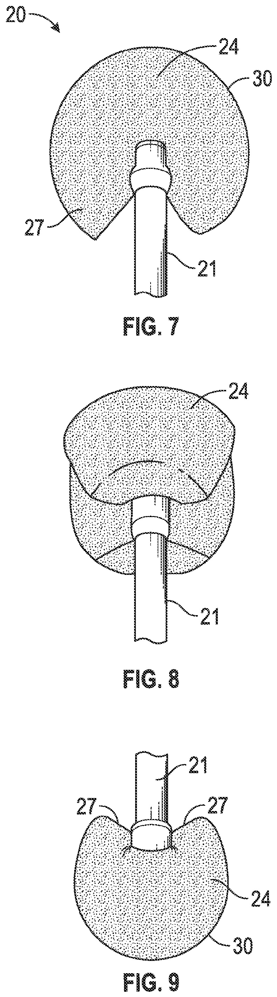

FIGS. 1, 2 and 4 to 6 illustrate the preparation sponge device 20 from different directions, with the sponge 24 in a dry, unexpanded state. When the sponge is wetted, the sponge material swells and continues to swell until it creates a rounded ball-like or substantially spherical shape, as illustrated in FIGS. 7 to 9. The handle attachment at the center of the sponge restricts the central portion from swelling, while allowing the remainder of the sponge to swell or expand freely, creating eye preparation surfaces 30 which are all rounded when the sponge is sufficiently wetted, as seen in FIGS. 7 to 9. Because the sponge comprises more than half of a circle and the straight edge 27 of the sponge faces towards the handle when the device is in use, the straight edges on each side of the attachment area are moved away from the treatment area as the preparation sponge is wetted and expands, forming a generally V-shape as seen in FIG. 7 and 9. This moves edge surfaces away from the treatment area and avoids the risk of an edge surface contacting the eye.

In some embodiments, the sponge 24 is pre-wetted prior to use so that it is in the expanded condition of FIG. 7 to 9 before contacting the eye. Once wetted, the material has a softness and roundness that reduces the risk of causing epithelial defects when rubbing gently over the epithelial surface of the eye, as illustrated in FIG. 10. In other embodiments, the sponge may not be pre-wetted prior to use, but starts swelling and rounding up when touched to the tear meniscus from contact with eye fluids, so that it softens prior to the eye surface preparation procedure.

In some embodiments, rubbing or buffing the epithelium gently with the expanded sponge 24 removes lipids, mucus and microvilli as well as dead epithelial cells which would otherwise resist penetration of fluid through the epithelium. In some embodiments, the wet sponge is rubbed gently over the surface of the eye, for example in a circular pattern. In some embodiments, a topical anesthetic is applied to the eye before the application and use of the wet sponge.

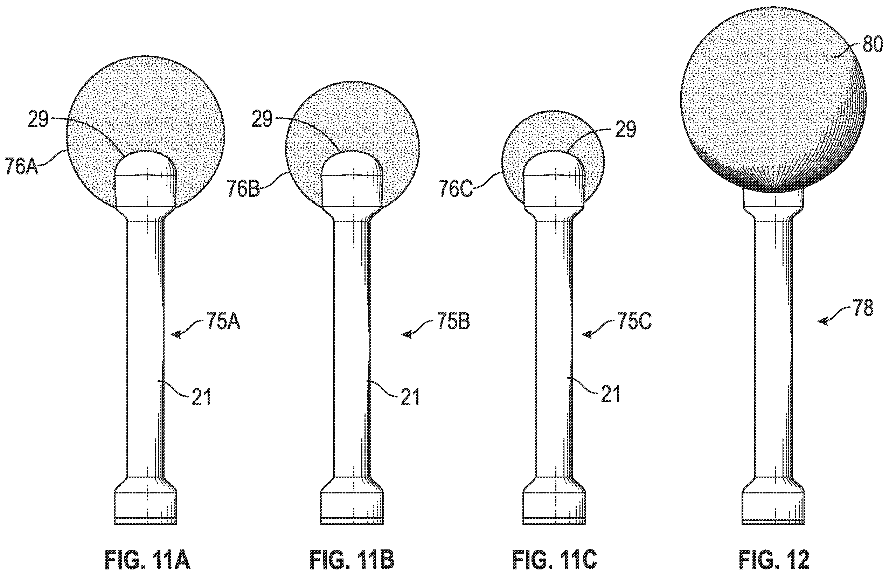

FIGS. 11A to 11C illustrate modified sponge devices 75A, 75B and 75C, respectively, which are similar to the sponge device of FIGS. 1 to 9, but have a completely circular head 76A, 76B, 76C attached to handle 21 instead of a part circular head with a straight lower edge as in the first embodiment. In some embodiments, the head diameters in FIGS. 11A to 11C are around 8 mm, 6.5 mm, and 4.5 mm, respectively, and the dry sponge head has flat opposite faces as in the first embodiments. When wetted, the sponge heads of FIGS. 11A to 11C also expand to a substantially spherical shape. Modified sponge device 78 of FIG. 12 has a spherical head 80 of expanded foam material. This device is ready to use with a completely rounded surface, and absorbs liquid at a faster rate than the dry, compressed foam heads of the previous embodiments. Head 80 may be made of urethane sponge material, for example. This sponge device maintains structural rigidity for preparation of the epithelium for penetration of riboflavin solutions or other solutions or medications.

Any suitable sponge shape or material is contemplated for use in manipulation of tissue on the surface of an eye, including the sponge head shape of the first embodiment (FIG. 1 to 10 ) as well as the alternative sponge head shapes of FIG. 11A to 12 , as well as other shapes. In alternative embodiments, the preparation sponge device may be of more oval, Q-tip like shape, or may comprise a sponge or instrument wipe wrapped around a finger. However, the sponge head shape of FIG. 1 to 10 is found to work well in reducing the risk of causing injury or epi-defects while improving delivery of ophthalmic solution to the cornea, due to the generally rounded shape as well as the elimination of any sharp edges in the eye preparation surface portion when the sponge is wetted and expands as seen in FIG. 7 to 9. The sponge heads of FIG. 11A to 11C will also expand to a rounded, partially or completely spherical shape on wetting. The spherical sponge head of FIG. 12 is already completely expanded and rounded prior to use and absorbs liquid at a faster wicking rate than the heads of the other embodiments which are of dry compressed foam. The sponge heads in any of the preceding embodiments may be pre-wetted either prior to application to the eye or by placing on the eye surface to absorb tears, after which the head tends to swell or expand to a more rounded off shape with no sharp edges in the partially spherical tissue preparation surface.

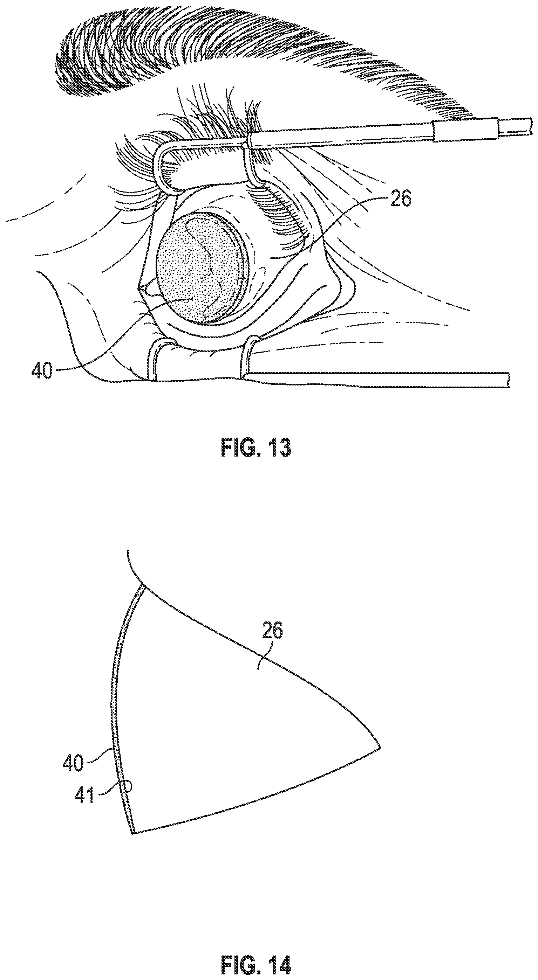

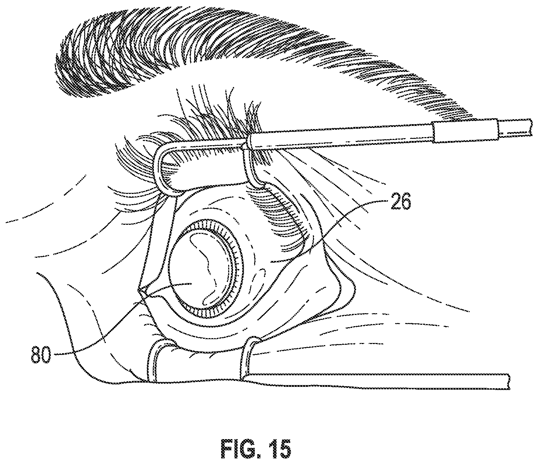

Although FIGS. 13 and 15 illustrate the eye being held open by a speculum during the solution loading procedure, which does not form part of the invention, this is often not necessary either for the eye preparation step of FIG. 12 or the loading procedure using sponge 40 or 80. In fact, the surgeon may hold the eye open manually during these procedures, or in some cases the patient may simply try not to blink.

The ophthalmic solution is dripped onto the holding or loading sponge while it is placed over the eye, or the sponge is pre-soaked with the ophthalmic solution, or both, but does not form part of the invention. The second, loading sponge, for example sponge 40 or 80, which does not form part of the invention, acts as a reservoir to hold the solution against the eye surface and to allow application of additional solution. Simply dropping ophthalmic solution directly onto the eye surface or placing drops onto the eye results in the drops running off the eye, and has a limited effect. In contrast, the solution holding sponge, which does not form part of the invention, places ophthalmic solution directly against the surface of the eye in a location where treatment is needed over an extended time period, allowing more efficient penetration into the cornea. The ophthalmic solution is a riboflavin solution of 0.2% to 10.0% by weight riboflavin in an aqueous carrier, and optionally, sodium iodide, catalase, artificial tear solution or any combination thereof. If the sponge is pre-loaded with riboflavin, it is stored in a dark packaging material prior to use to avoid or reduce light degradation. The riboflavin solution contains other additives for increased cross-linking, for example additives as described in PCT Application Publication No. WO 2013/148896. The sponge is round, but the sponge may be of other shapes (such as oval or eye shaped). The solution holding or loading sponge operatively covers all or a portion of the eye or the cornea. The holding sponge, which does not form part of the invention, is made from any suitable sponge material such as cellulose or any fast wicking, lint-free material such as polyvinyl acetate, for example any of the materials described above in connection with the manipulation or preparation sponge devices of FIGS. 1 to 12. The sponge material of the preparation sponge 20 in one embodiment was PVA sponge material with a pore size in the range of about 60 microns to about 1000 microns. In one embodiment, the pore size of the preparation sponge 20 was in the range from about 60 to about 120 microns. In one example, the pore size was about 60 microns. The holding or loading sponge, which does not form part of the invention, may have a larger pore size than the preparation sponge 24 in order to hold more liquid for corneal loading purposes.

Where the preparation sponge is a round sponge, the sponge operatively covers all or a portion of the eye or the cornea. In some embodiments, the size of the preparation sponge does not exceed the size of the eye or the cornea. In some embodiments, the diameter of the sponge is about 4.5 mm to about 8 mm. In some embodiments, the sponge is about 1 mm in thickness. A series of different diameter loading sponges may be provided for selection by the physician depending on the desired treatment area.

The above sponges may be used in the two stage method described above for enhanced, faster penetration of any ocular treatment solution through the epithelium and into the cornea, while reducing or minimizing the risk of epithelial defects. The ophthalmic solution may be an ocular riboflavin solution for use in corneal treatment such as photochemical cross linking, or for other ophthalmic uses. In one embodiment, the riboflavin solution contains 0.1 wt. % to 5.0 wt. % riboflavin in an aqueous carrier solution, or up to 10.0% in some cases. In some embodiments, the solution contains about 0.5 wt. % riboflavin. In other examples, the solution contains about 1.0 wt. % riboflavin or about 2.0 wt. % riboflavin. The higher concentration of riboflavin can increase corneal cross-linking if associated with higher amounts of oxygen in the cornea. The riboflavin solution may be stored in an actinic glass or UV and visible light protected plastic containers prior to use, to avoid activation of the riboflavin by ambient light.

The above preparation sponge is designed to increase permeability of the epithelium layer with low or minimal epithelial defects resulting from the preparation or tissue manipulation step. Results of testing show that use of the sponge of FIGS. 1 to 9 with a part-circular edge, which expands to a part-spherical or ball-like shape when wetted, may eliminate or substantially eliminate epithelial defects. Similar results may be achieved with the sponges of FIGS. 11A to 11C with a completely circular edges, which also expand to a completely or partially spherical shape, as well as the sponge device of FIG. 12, which is pre-expanded to a spherical shape. The preparation sponge of FIGS. 1 to 9 and the loading or holding sponge device of FIGS. 13 to 15, which do not form part of the invention, have also been found to significantly reduce the time needed for sufficient riboflavin solution to penetrate into and saturate the cornea, as compared to the time needed with no pre-treatment of the epithelium to increase permeability. Some examples are provided below.

### Example

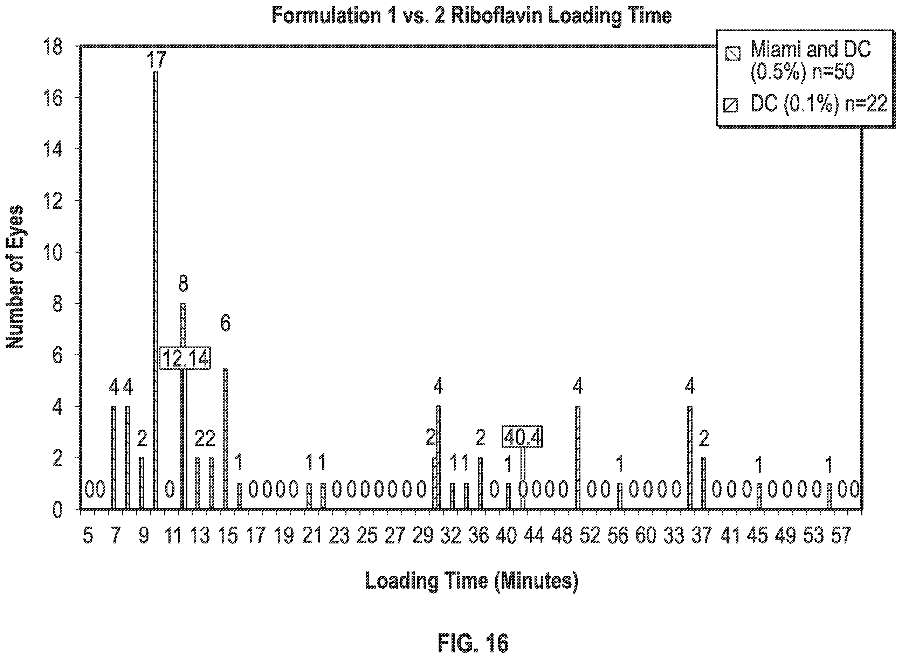

Two formulations of riboflavin solution were tested to determine saturation or riboflavin solution loading time after pre-treatment of the epithelium using the preparation sponge device of the above embodiments. Formulation 1 had a riboflavin concentration of 0.1%. Formulation 2 had a riboflavin concentration of 0.5%. Results are compared in FIG. 16.

Saturation was determined using "serial slit-lamp assessments" of the cornea at approximately 5 minute intervals. Riboflavin has a characteristic green color when illuminated with visible light. Slit-lamp assessments using visible light reveal the depth and uniformity of riboflavin throughout the corneal thickness.

### Inclusion Criteria:

Patients who had undergone trans-epithelial cross-linking in one or both eyes were included in the analysis. Patients with a diagnosis of keratoconus or post-LASIK ectasia were included in this analysis.

### Exclusion Criteria:

Patients with previous RK, INTACS, more than one cross-linking procedure per eye, and/or patients who were pseudo-phakic or had a diagnosis of nuclear sclerotic cataract were excluded from this analysis

### Results - Formulation 1, 0.1% Riboflavin, Formulation 2, 0.5% Riboflavin

The results are shown in FIG. 16. Trans epithelial loading time using one of the loading sponges described above with formulation 1 ranged from 30 minutes minimum to 56 minutes maximum, with an average loading time of 40.4 minutes. Trans epithelial loading time with formulation 2 ranged from 7 to 30 minutes with an average loading time of 12.14 minutes.

The time period for good, homogeneous loading of riboflavin solution into the cornea, i.e. trans epithelial riboflavin loading time, was about 10 to 30 minutes using the delivery augmentation and delivery methods described above. Corresponding loading times without removal or pre-treatment of the epithelium can be up to three hours, significantly adding to the overall time for completion of a corneal treatment procedure.

According to the invention, there is provided a preparation sponge for use in manipulation of tissue on the surface of an eye, comprising:
a tissue preparation surface, shaped for manipulating and rubbing across the surface of an eye, wherein at least the tissue preparation surface of the sponge is rounded; and has no sharp edges.

Also disclosed is the preparation sponge of the invention, wherein the head is of spherical shape.

Also disclosed is the preparation sponge of the invention, wherein the thickness of the sponge is about 1 mm.

Also disclosed is the preparation sponge of the invention, wherein the diameter of the sponge is in the range from about 4.5 mm. to about 8 mm.

Also disclosed is the preparation sponge of the invention, wherein the sponge material comprises cellulose or polyvinyl acetate, or urethane sponge material.

Also disclosed is the preparation sponge of the invention, wherein the PVA sponge material has a pore size in the range from 60 to 1000 microns.

Also disclosed is the preparation sponge of the invention, wherein the sponge is pre-wetted with an ophthalmic composition.

Also disclosed is the preparation sponge of the invention, wherein the preparation sponge further comprises a handle operatively connected to the preparation sponge.

Also disclosed is the preparation sponge of the invention, wherein the ophthalmic solution comprises a riboflavin composition, artificial tears, or a combination thereof.

## Claims

1. A preparation sponge for use in manipulation of tissue on the surface of an eye comprising:
a tissue preparation surface, shaped for manipulating and rubbing across the surface of an eye; wherein at least the tissue preparation surface of the sponge is rounded; and has no sharp edges.

2. A preparation sponge according to Claim 1, wherein the sponge is circular or part-circular shape when dry and is configured to absorb liquid and expand into a spherical or part-spherical shape when wetted.

3. A preparation sponge according to Claim 1 or 2, wherein the sponge is pre-wetted with an ophthalmic composition.

4. A preparation sponge according to Claim 3, wherein the ophthalmic composition is selected from a riboflavin solution, artificial tears, or a combination thereof.

5. A preparation sponge according to Claim 4, wherein the riboflavin solution contains about 0.5 wt. % riboflavin, about 1.0 wt. % riboflavin, or about 2.0 wt. % riboflavin.

6. A preparation sponge according to Claim 4, wherein the riboflavin solution contains 0.1 wt. % to 5.0 wt. %; or 0.1 wt. % to 10.0 wt. % riboflavin in an aqueous carrier solution.

7. A preparation sponge according to Claim 4, wherein the ophthalmic solution is 0.2% to 10.0% by weight riboflavin in an aqueous carrier and sodium iodide, catalase, artificial tears, or any combinations thereof.

8. A preparation sponge according to any one of Claims 1-7, wherein the sponge is made of a cellulose sponge material.

9. A preparation sponge according to Claim 8, wherein the cellulose sponge material is selected from a cellulose, polyvinyl acetate (PVA), or urethane sponge material.

10. A preparation sponge according to Claim 9, wherein the PVA sponge material has a pore size in the range of about 60 micrometers (microns) to about 1000 micrometers (microns).

11. A preparation sponge according to any one of Claims 1-10, wherein the preparation sponge further comprises a handle operatively connected to the preparation sponge.

12. A preparation sponge according to Claim 11, wherein the preparation sponge is made of a relatively rigid, highly absorbent natural cellulose or highly absorbent, fast wicking polyvinyl acetate (PVA) material.

13. A preparation sponge according to Claim 11 or 12, wherein the thickness of the sponge is around 1 mm.

14. A preparation sponge according to any one of Claims 11-13, wherein the diameter of the sponge is in the range from 4.5 mm to around 8 mm.

15. A preparation sponge according to any one of Claims 1-14, wherein the eye preparation surfaces are all rounded when the sponge is sufficiently wetted.

## Patentansprüche

1. Präparationsschwamm zur Verwendung bei der Manipulation von Gewebe auf der Oberfläche eines Auges, umfassend: eine Gewebepräparationsoberfläche, die zum Manipulieren und Reiben über die Oberfläche eines Auges geformt ist; wobei zumindest die Gewebepräparationsoberfläche des Schwamms abgerundet ist; und keine scharfen Kanten aufweist.

2. Präparationsschwamm nach Anspruch 1, wobei der Schwamm im trockenen Zustand eine kreisförmige oder teilweise kreisförmige Form hat und so konfiguriert ist, dass er Flüssigkeit aufnimmt und sich bei Benetzung zu einer kugelförmigen oder teilweise kugelförmigen Form ausdehnt.

3. Präparationsschwamm nach Anspruch 1 oder 2, wobei der Schwamm mit einer ophthalmischen Zusammensetzung vorbenetzt ist.

4. Präparationsschwamm nach Anspruch 3, wobei die ophthalmische Zusammensetzung aus einer Riboflavinlösung, künstlichen Tränen oder einer Kombination davon ausgewählt ist.

5. Präparationsschwamm nach Anspruch 4, wobei die Riboflavinlösung etwa 0,5 Gew.-% Riboflavin, etwa 1,0 Gew.-% Riboflavin oder etwa 2,0 Gew.-% Riboflavin enthält. Riboflavin enthält.

6. Präparationsschwamm nach Anspruch 4, wobei die Riboflavinlösung 0,1 Gew.-% bis 5,0 Gew.-%; oder 0,1 Gew.-% bis 10,0 Gew.-% Riboflavin in einer wässrigen Trägerlösung enthält.

7. Präparationsschwamm nach Anspruch 4, wobei die ophthalmische Lösung 0,2 bis 10,0 Gew.-% Riboflavin in einem wässrigen Träger und Natriumiodid, Katalase, künstliche Tränen oder beliebige Kombinationen davon enthält.

8. Präparationsschwamm nach einem der Ansprüche 1-7, wobei der Schwamm aus einem Zelluloseschwamm-Material hergestellt ist.

9. Präparationsschwamm nach Anspruch 8, wobei das Zelluloseschwammmaterial aus einem Zellulose-, Polyvinylacetat- (PVA)- oder Urethanschwammmaterial ausgewählt ist.

10. Präparationsschwamm nach Anspruch 9, wobei das PVA-Schwammmaterial eine Porengröße im Bereich von etwa 60 Mikrometern (Mikron) bis etwa 1000 Mikrometern (Mikron) aufweist.

11. Präparationsschwamm nach einem der Ansprüche 1-10, wobei der Präparationsschwamm weiter einen Griff umfasst, der funktionsfähig mit dem Präparationsschwamm verbunden ist.

12. Präparationsschwamm nach Anspruch 11, wobei der Präparationsschwamm aus einem relativ steifen, stark saugfähigen natürlichen Zellulosematerial oder einem stark saugfähigen, schnell trocknenden Polyvinylacetat- (PVA)- Material hergestellt ist.

13. Präparationsschwamm nach Anspruch 11 oder 12, wobei die Dicke des Schwamms etwa 1 mm beträgt.

14. Präparationsschwamm nach einem der Ansprüche 11-13, wobei der Durchmesser des Schwamms im Bereich von 4,5 mm bis etwa 8 mm liegt.

15. Präparationsschwamm nach einem der Ansprüche 1-14, wobei die Augenpräparationsflächen alle abgerundet sind, wenn der Schwamm ausreichend benetzt ist.

## Revendications

1. Éponge de préparation pour une utilisation dans la manipulation de tissus à la surface d'un œil comprenant : une surface de préparation tissulaire, formée pour être manipulée et frottée sur la surface d'un oeil ; dans laquelle au moins la surface de préparation tissulaire de l'éponge est arrondie ; et ne présente pas de bords tranchants.

2. Éponge de préparation selon la revendication 1, dans laquelle l'éponge est de forme circulaire ou partiellement circulaire lorsqu'elle est sèche et est configurée pour absorber du liquide et se dilater en une forme sphérique ou partiellement sphérique lorsqu'elle est mouillée.

3. Éponge de préparation selon la revendication 1 ou la revendication 2, dans laquelle l'éponge est pré-mouillée avec une composition ophtalmique.

4. Éponge de préparation selon la revendication 3, dans laquelle la composition ophtalmique est choisie parmi une solution de riboflavine, des larmes artificielles, ou une de leurs combinaisons.

5. Éponge de préparation selon la revendication 4, dans laquelle la solution de riboflavine contient environ 0,5 % en poids de riboflavine, environ 1,0 % en poids de riboflavine, ou environ 2,0 % en poids de riboflavine.

6. Éponge de préparation selon la revendication 4, dans laquelle la solution de riboflavine contient 0,1 % en poids à 5,0 % en poids ; ou 0,1 % en poids à 10,0 % en poids de riboflavine dans une solution porteuse aqueuse.

7. Éponge de préparation selon la revendication 4, dans laquelle la solution ophtalmique est de 0,2 % à 10,0 % en poids de riboflavine dans un support aqueux et d'iodure de sodium, de catalase, de larmes artificielles, ou toute combinaison de ceux-ci.

8. Éponge de préparation selon l'une quelconque des revendications 1 à 7, dans laquelle l'éponge est constituée d'un matériau d'éponge de cellulose.

9. Éponge de préparation selon la revendication 8, dans laquelle le matériau d'éponge de cellulose est choisi parmi un matériau d'éponge de cellulose, de polyacétate de vinyle (PVA) ou d'uréthane.

10. Éponge de préparation selon la revendication 9, dans laquelle le matériau d'éponge en PVA présente une taille de pore dans la plage d'environ 60 micromètres (microns) à environ 1000 micromètres (microns).

11. Éponge de préparation selon l'une quelconque des revendications 1 à 10, dans laquelle l'éponge de préparation comprend en outre une poignée reliée de manière opérationnelle à l'éponge de préparation.

12. Éponge de préparation selon la revendication 11, dans laquelle l'éponge de préparation est constituée d'une cellulose naturelle relativement rigide, hautement absorbante ou d'un matériau en polyacétate de vinyle (PVA) hautement absorbant, à absorption rapide.

13. Éponge de préparation selon la revendication 11 ou la revendication 12, dans laquelle l'épaisseur de l'éponge est d'environ 1 mm.

14. Éponge de préparation selon l'une quelconque des revendications 11 à 13, dans laquelle le diamètre de l'éponge est dans la plage de 4,5 mm à environ 8 mm.

15. Éponge de préparation selon l'une quelconque des revendications 1 à 14, dans laquelle les surfaces de préparation oculaire sont toutes arrondies lorsque l'éponge est suffisamment mouillée.
